# FASCICULE DE BREVET EUROPEEN

(11) **EP 2 139 910 B1**
(45) Date de publication et mention de la délivrance du brevet: **25.05.2011**
(21) Numéro de dépôt: 08718144.2
(22) Date de dépôt: 21.03.2008
(51) Int. Cl.: C07K 1/06, C07K 5/075, C07K 5/06, C07K 5/062, C07K 5/065

(54) **PROCEDE DE SYNTHESE DE PEPTIDES SANS SOLVANT**
VERFAHREN ZUR SYNTHESE VON PEPTIDEN OHNE LÖSUNGSMITTEL
METHOD FOR THE SYNTHESIS OF PEPTIDES WITHOUT SOLVENT

(30) Priorité: 21.03.2007 FR 0753970
(43) Date de publication de la demande: 06.01.2010
(73) Titulaire: Centre National de la Recherche Scientifique (CNRS), 75016 Paris (FR); Université de Montpellier I, 34006 Montpellier (FR)
(72) Inventeur: MARTINEZ, Jean, F-34720 Caux (FR); LAMATY, Frédéric, F-34000 Montpellier (FR); DECLERCK, Valérie, F-11300 Villelongue d'Aude (FR)
(74) Mandataire: Warcoin, Jacques
(86) Numéro de dépôt international: PCT/EP2008/053444
(87) Numéro de publication internationale: WO 2008/125418

(56) Documents cités:
- WO-A-2005/021517
- FULLER W D ET AL: "Urethane-protected alpha-amino acid N-carboxyanhydrides and peptide synthesis" BIOPOLYMERS, NEW YORK, NY, US, vol. 40, no. 2, 1 janvier 1996 (1996-01-01), pages 183-205, XP002278328 ISSN: 0006-3525
- MCCLUSKEY A ET AL: "Green chemistry approaches to the Knoevenagel condensation: comparison of ethanol, water and solvent free (dry grind) approaches" TETRAHEDRON LETTERS, ELSEVIER, AMSTERDAM, vol. 43, no. 17, 22 avril 2002 (2002-04-22), pages 3117-3120, XP004347873 ISSN: 0040-4039

## Description

La présente invention concerne un procédé de synthèse de peptides sans solvant.

Les peptides sont à présent considérés comme des principes actifs pharmaceutiques, du fait de leur indice thérapeutique élevé et de leur faible toxicité. En raison du développement de nouveaux systèmes d'administration accroissant leur biodisponibilité, on s'attend à ce que le marché des peptides thérapeutiques se développe rapidement au cours des prochaines années.

Mais il existe toujours un besoin de méthodes de synthèse efficaces pour ces composés. En dépit des procédures de production bien établies (en solution, en phase solide, par recombinaison) il subsiste encore de nombreux problèmes de développement associés à l'énorme quantité de solvant nécessaire à la synthèse, notamment sur supports solides (2000 à 5000 kg pour un gros peptide).

Le domaine de la « chimie verte » prend une ampleur grandissante en raison de la gravité des problèmes environnementaux actuels. Plusieurs domaines de cette discipline ont émergé : l'utilisation d'une matière première alternative et d'un réactif non toxique, l'emploi de processus naturels, l'utilisation de solvants alternatifs, la conception de produits chimiques plus sûrs, le développement d'autres conditions de réaction, la minimisation de la consommation énergétique...

Un domaine particulièrement actif repose sur l'utilisation de solvants alternatifs tels que des liquides aqueux, ioniques, fluorés ou supercritiques, pour remplacer les solvants organiques ou chlorés volatils et afin de résoudre les problèmes de traitement ou de recyclage des déchets à base de solvants.

Une approche alternative consiste à réaliser des réactions chimiques en l'absence de solvant. Des techniques telles que le mélange ou le broyage de solides se sont avérées efficaces. Néanmoins, aucune application de ces techniques à des domaines tels que la synthèse des peptides ou d'aminoacides n'a été entreprise, en dépit de l'importance de ces biomolécules.

Les auteurs de la présente invention ont découvert de manière surprenante de nouvelles voies de synthèse de peptides dans des conditions sans solvant. L'article de Fuller et al (Biopolymers, 40(2), pages 183-205) décrit un procédé de synthèse d'un peptide qui consiste à faire réagir un carboxyanhydride d'aminoacide avec un aminoacide ou peptide en présence d'une base (NMM) et avec solvant.

De manière surprenante, les auteurs ont réalisé le couplage de carboxyanhydrides d'aminoacides protégés par un uréthane (UNCA) avec des aminoacides ou aminoesters, alors que tous ces composés sont restés sous leur forme solide, dans des conditions de broyage à billes et à température ambiante.

Plus précisément, l'invention concerne un procédé de synthèse d'un composé de formule (I) suivante : 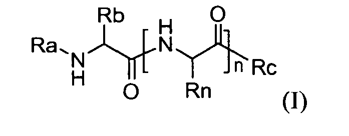 dans laquelle :
- n est un entier supérieur ou égal à 1, avantageusement compris entre 1 et 100, plus avantageusement compris entre 1 et 50, plus avantageusement encore égal à 1 ou 2 ;
- Rb et chaque Rn représentent indépendamment les uns des autres un atome d'hydrogène, un groupe aryle(alkyle en C₁ à C₆) ou un groupe alkyle en C₁ à C₆ substitué ou non par un groupe aryle, -COOH, -COO-(alkyle en C₁ à C₆), -CONH₂, - SH, hétéroaryle, -NH₂, -NHC(NH)(NH₂), -S-(alkyle en C₁ à C₆), -OH ou phénol dont les groupes -COOH, NH₂, OH, SH et NH sont éventuellement protégés par un ou plusieurs groupes N-protecteur ou O-protecteur identiques ou différents et différent du groupe Ra, avantageusement ce ou ces groupes N-protecteur et /ou O-protecteur sont stables dans les conditions d'élimination du groupe Ra ;
- Ra représente un groupe N-protecteur ;
- Rc représente un groupe -ORd dans lequel Rd représente un groupe alkyle en C₁ à C₆ ou un groupe -NReRf dans lequel Re et Rf représentent indépendamment l'un de l'autre un groupe N-protecteur,
caractérisé en ce qu'il comprend une étape (a) consistant à faire réagir en présence d'une base et sans solvant le composé de formule (II) suivante : 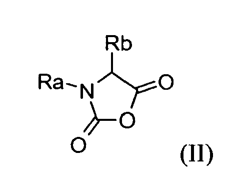 dans laquelle Ra et Rb sont tels que définis précédemment, avec le composé de formule (III) suivante : 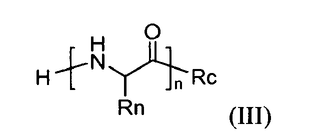 dans laquelle n, Rn et Rc sont tels que définis précédemment, ainsi que ses sels pharmaceutiquement acceptables, de préférence les chlorures, acétates et trifluoroacétates.

Au sens de la présente invention on entend par le terme « sans solvant », le fait que la réaction a lieu en absence de solvant. Un solvant selon l'invention est un produit qui solubilise les réactifs mais ne participe pas directement à la réaction. Ainsi, dans le cadre de la présente invention, la base n'est pas un solvant.

Par ailleurs, dans le procédé selon l'invention tous les réactifs utilisés sont à l'état solide. C'est en particulier le cas des composés de formule II et III et de la base. Ainsi, cette réaction a lieu à l'état solide, et non en solution. Avantageusement ces réactifs sont sous une forme solide finement divisée, tels qu'obtenue par un broyage à bille.
L'avantage de ce type de réaction est de supprimer l'utilisation de solvant (chimie verte) mais aussi de faciliter la mise en oeuvre de la réaction, le traitement et de permettre l'obtention de produits très purs.

Par le terme « groupe alkyle en C₁ à C₆ », on entend au sens de la présente invention tout groupe alkyle de 1 à 6 atomes de carbones, linéaire ou ramifié, en particulier, les groupes méthyle, éthyle, *n*-propyle, *iso*-propyle, *n*-butyle, *iso*-butyle, *sec-*butyle, *t*-butyle, *n*-pentyle, *n*-hexyle. Avantageusement il s'agit d'un groupe méthyle ou t-butyle.

Par le terme « groupe aryle », on entend au sens de la présente invention un ou plusieurs cycles aromatiques ayant 5 à 8 atomes de carbones, pouvant être accolés ou fusionnés. En particulier, les groupes aryles peuvent être des groupes monocycliques ou bicycliques, de préférence phényle, naphthyle, tetrahydronaphthyle ou indanyle. Avantageusement il s'agit d'un groupe phényle.

Par le terme « groupe hétéroaryle », on entend au sens de la présente invention tout groupe aromatique hydrocarboné de 3 à 9 atomes contenant un ou deux hétéroatomes, tels que par exemple des atomes de soufre, azote ou oxygène. L'hétéroaryle selon la présente invention peut être constitué par un ou deux cycles fusionnés ou accolés. Des exemples de groupes hétéroaryle sont les groupes furyle, isoxazyle, pyridyle, pyrimidyle, benzimidazole, benzoxazole, benzothiazole.

Par le terme « groupe N-protecteur », on entend au sens de la présente invention tout substituant qui protège le groupe NH₂ contre les réactions indésirables tels que les groupes N-protecteur décrits dans Greene, "Protective Groups In Organic Synthesis", (John Wiley & Sons, New York (1981)) et Harrison et al. « Compendium of Synthetic Organic Methods", Vols. 1 à 8 (J. Wiley & sons, 1971 à 1996). Les groupes N-protecteur comprennent les carbamates, amides, dérivés N-alkylés, dérivés amino acétal, dérivés N-benzylés, dérivés imine, dérivés énamine et dérivés N-hétéroatome. En particulier, le groupe N-protecteur comprend le formyle, l'acétyle, le benzoyle, le pivaloyle, le phénylsulfonyle, le benzyle (Bn), le t-butyloxycarbonyle (boc), le benzyloxycarbonyle (cbz), le *p-*méthoxybenzyloxycarbonyle, le *p*-nitrobenzyl-oxycarbonyle, le trichloroéthoxycarbonyle (troc), l'allyloxycarbonyle (alloc), lé 9-fluorénylméthyloxycarbonyle (fmoc), le trifluoroacétyle ou les carbamates de benzyle (substitués ou non). Il est avantageux d'utiliser soit du boc soit du cbz en tant que groupe N-protecteur en raison de la facilité relative d'élimination, par exemple par des acides modérés dans le cas du boc, par exemple l'acide trifluoroacétique ou l'acide chlorhydrique dans de l'acétate d'éthyle ; ou par une hydrogénation catalytique dans le cas de cbz. Avantageusement, il s'agit du groupe boc.

Par le terme "groupe O-protecteur", on entend au sens de la présente invention tout substituant qui protège le groupe hydroxyle ou carboxyle, c'est à dire un atome d'oxygène réactif, contre les réactions indésirables tels que les groupes O-protecteur décrits dans Greene, "Protective Groups In Organic synthesis", (John Wiley & Sons, New York (1981)) et Harrison et al. « Compendium of Synthetic Organic Methods", Vols. 1 à 8 (J. Wiley & sons, 1971 à 1996). Les groupes O-protecteur comprennent les éthers de méthyle ou d'alkyle substitués ou non, par exemple, méthoxyméthyle, benzyloxyméthyle, 2-méthoxyéthoxyméthyle, 2-(triméthylsilyle) éthoxyméthyle, t-butyle, benzyle et triphénylméthyle, les éthers de benzyle (substitués ou non), les tétrahydropyranyle éthers, les éthers d'allyle, les éthyle éthers substitués, par exemple, 2,2,2-trichloroéthyle, les silyle éthers ou les éthers d'alkylsilyle, par exemple, triméthylsilyle, t-butyldiméthylsilyle et t-butyldiphénylsilyle, les éthers d'hétérocycle; et les esters préparés par réaction du groupe hydroxyle avec un acide carboxylique par exemple, les esters de tert-butyle, de benzyle ou de méthyle, les carbonates en particulier le carbonate de benzyle ou d'halogénoalkyle, l'acétate, le propionate ou le benzoate.
Avantageusement il s'agit du groupe benzyle.
Par la suite les composés de formule I peuvent être déprotégés de façon à obtenir les peptides dont les fonctions -OH, -NH₂, -SH, -NH et -COOH sont non protégés.

Dans un mode de réalisation avantageux de l'invention, n est égal à 1 et l'étape (a) consiste à faire réagir en présence d'une base et sans solvant le composé de formule (II) avec le composé de formule (III-1) suivante : 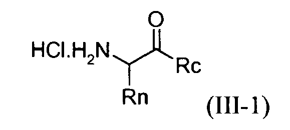 dans laquelle R1 et Rc sont tels que définis précédemment de façon à obtenir le composé de formule (I-1) suivante : 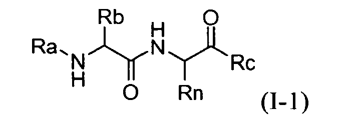 dans laquelle Ra, Rb, Rc et Rn sont tels que définis précédemment.

L'étape (a) est avantageusement effectuée au moyen d'un broyage à billes.

Il est particulièrement avantageux d'effectuer l'étape (a) à partir de produits de départ de formule (II) et (III) fraîchement préparés.

La base est une base solide et peut être avantageusement choisie dans le groupe constitué par les carbonates, de préférence les hydrogénocarbonates de sodium, de potassium et de césium, et les carbonates de sodium, de potassium et de césium. Avantageusement il s'agit de l'hydrogénocarbonate de sodium.

Les substituants Rb et chaque substituant Rn peuvent être choisis indépendamment dans le groupe constitué par l'hydrogène, -CH₃, le groupe benzyle, -CH₂CONH₂, -CHCH₃C₂H₅, -(CH₂)₃NHC(NH)(NH₂), -CH(OH)CH₃, -CH₂COOH, -CH₂SH, -CH₂CH₂COOH, -CH₂CH₂CONH₂, imidazolylméthyle, propyle, -CH₂CH(CH=)₂, -(CH₂)₄NH₂, -(CH₂)₂SCH₃, -CH₂OH, indol-2-ylméthyle, p-méthylphénol, isopropyle. Comme indiqué précédemment, les fonctions NH₂, NH, COOH, SH et OH de ces groupes sont avantageusement protégés par un ou plusieurs groupe O-protecteur et/ou N-protecteur identiques ou différents et différent de Ra. Avantageusement ce ou ces groupes N-protecteur et /ou O-protecteur sont stables dans les conditions d'élimination du groupe Ra.

Dans un mode de réalisation avantageux de l'invention :
- Ra, Re et Rf sont choisis indépendamment l'un de l'autre dans le groupe constitué par le *tert*-butyloxycarbonyle, le 9-fluorénylméthyloxycarbonyle, le benzyloxycarbonyle, le nitroveratryloxycarbonyle et/ou
- Rb est choisi dans le groupe constitué par l'isopropyle, le benzyle et
- CH₂COOt-Bu et/ou
- chaque Rn est choisi indépendamment des autres dans le groupe constitué par le méthyle, le benzyle et -CH₂CH(CH₃)₂ et/ou
- Rd est choisi dans le groupe constitué par le méthyle et le tertiobutyle.

Dans un mode de réalisation avantageux de l'invention, le composé (II) est choisi dans le groupe constitué par les composés de formules (II-a), (II-b) et (II-c) suivantes : 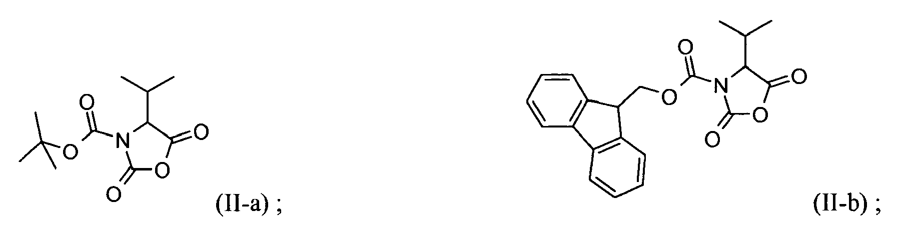 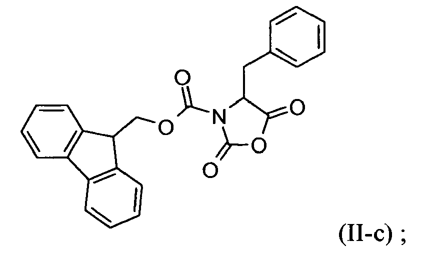
- le composé (III) est choisi dans le groupe constitué par les composés de formules (III-a), (III-b), (III-c), (III-d) et (III-e) suivantes :

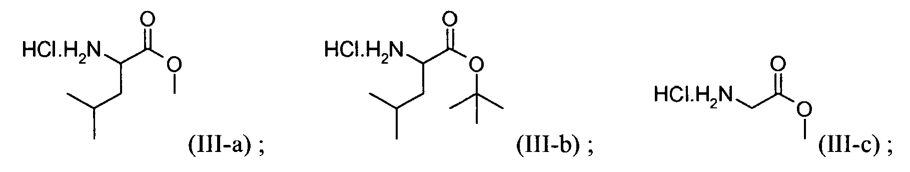

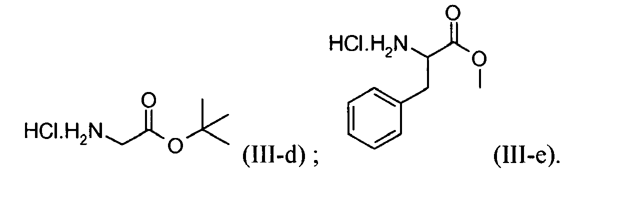

Dans un autre mode de réalisation avantageux de l'invention,
- le composé de formule (II) a la formule (II-2) suivante : 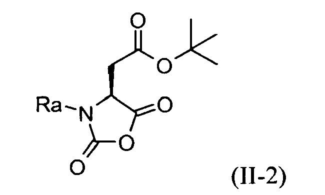 dans laquelle Ra est tel que défini précédemment,
- le composé de formule (III) a la formule (III-2) suivante : 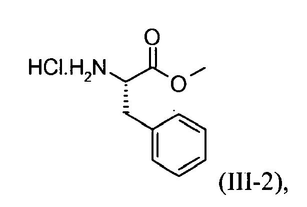 et le composé de formule (I) a la formule (I-2) suivante : 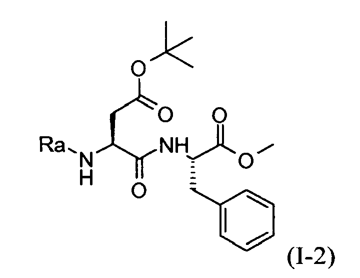 dans laquelle Ra est tel que défini précédemment.

Le composé de formule (II-2) peut être préparé par un procédé comprenant les étapes successives suivantes :
1) protection du groupe amine du composé de formule (IV) suivante : 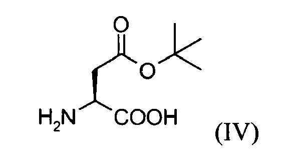 par un groupement N-protecteur Ra pour former le composé de formule (V) suivante 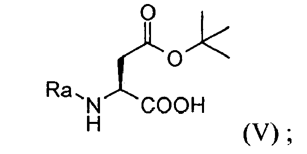
2) réaction du composé de formule (V) avec un halogénure de benzyle en présence d'une base, avantageusement le carbonate de césium, pour obtenir le composé de formule (VI) suivante : 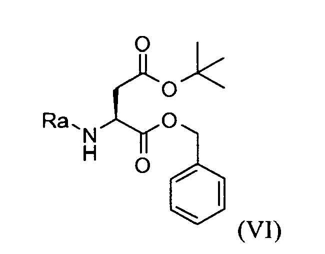 dans laquelle Ra est tel que défini ci-dessus ;
3) protection du groupe amine du composé de formule (VI) par un groupe N-protecteur Rg pour former le composé de formule (VII) suivante : 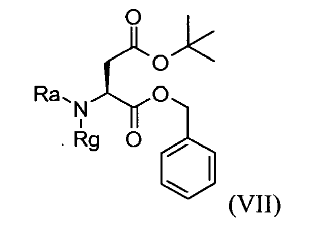 dans laquelle Ra et Rg sont tels que définis ci-dessus ;
4) réduction du composé de formule (VII) en composé de formule (VIII) suivante : 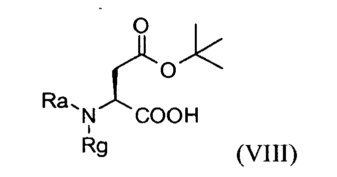 dans laquelle Ra et Rg sont tels que définis ci-dessus ;
5) cyclisation du composé de formule (VIII) en composé de formule (II-2) par réaction avec la DMF et le chlorure d'oxalyle.

Les substituants Ra et Rg représentent avantageusement le *tert-*butyloxycarbonyle.

Dans un mode de réalisation avantageux, le procédé comprend les étapes successives supplémentaires suivantes :
- (b) faire réagir le composé de formule (I-2) obtenu à l'étape (a) avec un acide, avantageusement l'acide chlorhydrique gazeux, pour former le composé de formule (I-3) suivante : 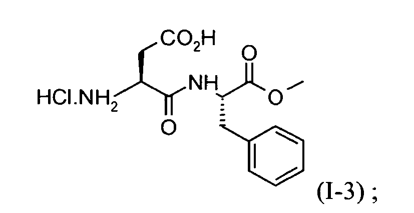
- (c) faire réagir le composé de formule (I-3) avec une base, avantageusement le bicarbonate de sodium, pour former le composé de formule (I-4) suivante :

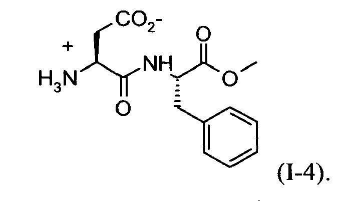

L'invention va maintenant être illustrée par les exemples suivants.

### Exemple 1 : Synthèse de dipeptides

Les inventeurs ont réalisé la réaction sans solvant entre un UNCA et un dérivé d'aminoacide pour former un dipeptide selon l'équation ci-dessous.

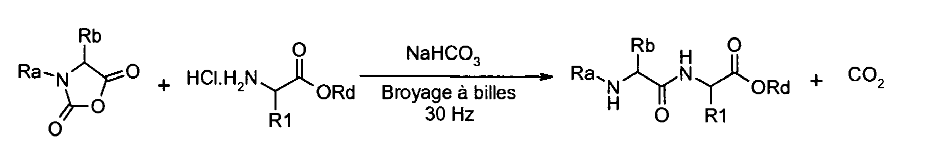

Cette réaction a été testée dans le couplage de Boc-Val-NCA, Fmoc-Val-NCA et Boc-Phe-NCA (1 éq.) avec divers acides aminés (1 éq.) en présence de NaHCO₃ (1,5 éq.) dans une cuve en acier trempé contenant des billes d'acier. La cuve a été agitée pendant 1 heure à une fréquence de 30 Hz. L'analyse du mélange réactionnel a détecté l'existence exclusive du dipeptide. Les résultats sont rapportés dans le tableau 1 avec divers UNCA et dérivés d'acides aminés.

**Tableau 1.**

| **UNCA** | **Acides aminés** | **Dipeptides** | **Conversion (%)** | **Rendement (%)** |
|---|---|---|---|---|
| Boc-Val-NCA | HCLH-Leu-OMe | Boc-Val-Leu-OMe | 100 | 87 |
| | HCLH-Leu-OBut | Boc-Val-Leu-OBu*t* | 97 | 85 |
| | HCl.H-Ala-OMe | Boc-Val-Ala-OMe | 100 | 100 |
| | HCl.H-Ala-OBu*t* | Boc-Val-Ala-OBu*t* | 100 | 100 |
| | HCl.H-Phe-OMe | Boc-Val-Phe-OMe | 100 | 88 |
| Fmoc-Val-NCA | HCl.H-Leu-OMe | Fmoc-Val-Leu-OMe | 90 | - |
| | HCl.H-Leu-O-But | Fmoc-Val-Leu-OBu*t* | 92 | - |
| | HCl.H-Ala-OMe | Fmoc-Val-Ala-OMe | 100 | 76 |
| | HCl.H-Ala-OBu*t* | Fmoc-Val-Ala-OBu*t* | 78 | - |
| | HCl.H-Phe-OMe | Fmoc-Val-Phe-OMe | 93 | - |
| Boc-Phe-NCA | HCl.H-Leu-O-Me | Boc-Phe-Leu-OMe | 85 | - |
| | HCl.H-Leu-OBut | Boc-Phe-Leu-OBu*t* | 100 | 70 |
| | HCl.H-Ala-OMe | Boc-Phe-Ala-OMe | 99 | 79 |
| | HCl.H-Ala-OBu*t* | Boc-Phe-Ala-OBu*t* | 100 | 73 |
| | HCl.H-Phe-OMe | Boc-Phe-Phe-OMe | 58 | - |

Les divers dérivés d'UNCA ne présentent pas le même profil de réactivité. Dans tous les cas, Boc-Val-NCA a été transformé quantitativement en vue de la formation du dipeptide tandis que Fmoc-Val-NCA dans les deux cas a donné une conversion légèrement inférieure.

Il convient de noter que les meilleurs résultats ont été obtenus avec une substance de départ fraîchement préparée, soit l'UNCA, soit l'aminoester. La réaction était sinon incomplète et l'hydrolyse de l'UNCA a été observée.

### Exemple 2 : Synthèse de l'aspartame.

L'aspartame, ou α-L-aspartyl-L-phénylalanine-méthyle-ester, est un édulcorant nutritif approximativement 150 fois plus intense que le saccharose. C'est un dipeptide commercialement attrayant qui n'a pourtant pas encore été préparé par le biais des UNCA, ni en présence, ni en l'absence de solvant.

Les inventeurs ont obtenu de l'aspartame protégé en une étape à partir de H-Phe-OMe.HCl (III-e) et de Boc-Asp(O-*t*-Bu)-NCA (II-a) par broyage à billes sans solvant.

Les groupes protecteurs Boc et *t*-Bu ont été choisis puisqu'ils peuvent être clivés en même temps dans des conditions acides. Afin d'éviter davantage l'utilisation de solvant, HCl gazeux a été utilisé pour éliminer les groupes protecteurs.

### 2.1. Préparation de Boc-Asp(O-tBu)-NCA (II-a)

Cette méthode a consisté en la cyclisation de l'acide carboxylique libre à chaîne principale avec l'un des Boc protégeant la fonction amine, nécessitant de ce fait tout d'abord la préparation de (Boc)₂-Asp(O-*t*-Bu)-OH (VII-a). Ceci a été réalisé par l'estérification de l'acide α-carboxylique de Boc-Asp(O-*t*-Bu)-OH (V-a) en ester benzylique (VI-a), suivie d'une réaction avec Boc₂O en présence de DMAP pour donner le composé aminé protégé par deux groupes boc (VII-a), puis d'une déprotection du groupe ester benzylique par hydrogénation pour donner (VIII-a).

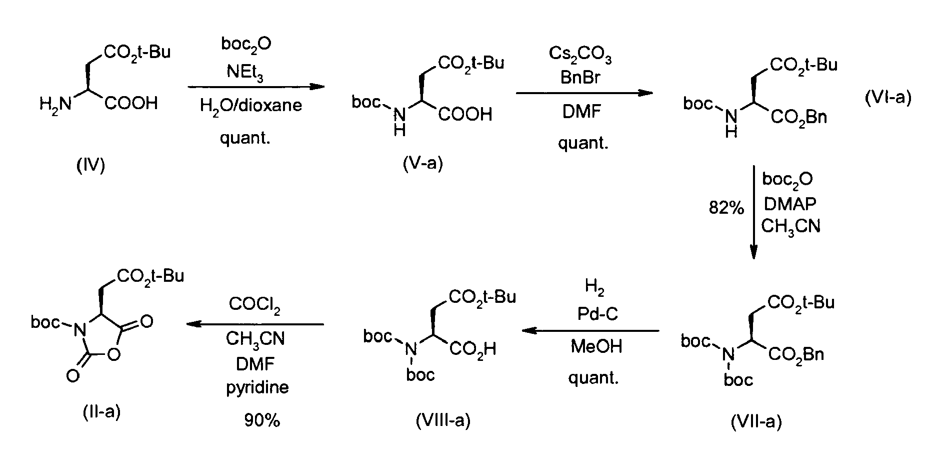

L'étape suivante a consisté en la cyclisation de l'aminoacide (VIII-a) protégé avec le sel de Vilsmeier. Les meilleurs résultats ont été obtenus en formant le sel avec de la DMF et du chlorure d'oxalyle dans de l'acétonitrile. Le composé (II-a) a été obtenu avec un rendement de 90 %.

### 2.2. Préparation de l'aspartame

La procédure décrite ci-dessus pour la préparation de dipeptides a été appliquée à la préparation de l'aspartame.

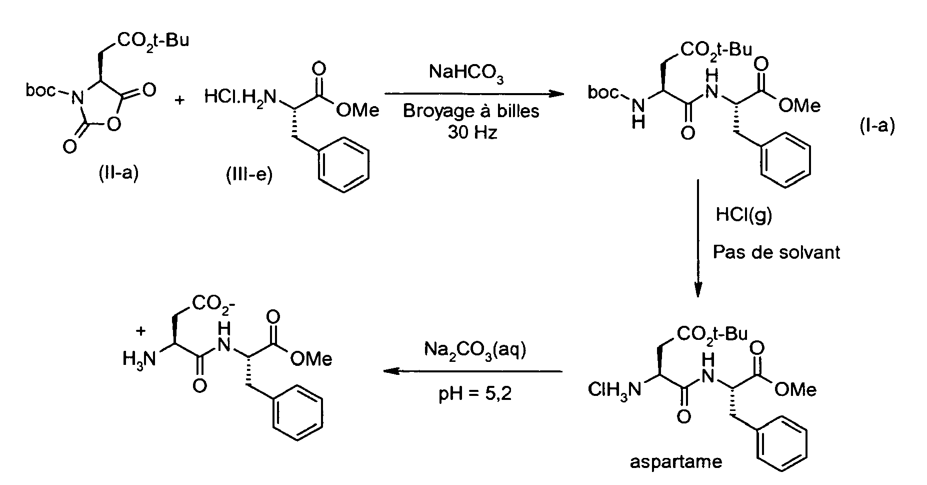

En partant de Boc-Asp(O-*t*-Bu)-NCA (II-a) et de H-Phe-OMe.HCl (III-e), le dipeptide (I-a) a été obtenu après 1 heure de broyage à billes.

Il a ensuite réagi directement avec HCl gazeux pendant 2 heures en l'absence de solvant pour éliminer les groupes protecteurs Boc and *t*-Bu et pour donner la forme chlorhydrate de l'aspartame. Notons que l'élimination des groupes protecteurs Boc et *t*-Bu n'a donné que des produits secondaires volatils. Il convient également de noter que le rendement des deux étapes était quantitatif.

Le chlorhydrate a été dissous dans l'eau et le pH a été ajusté à 5,2 avec une solution aqueuse de Na₂CO₃. L'aspartame obtenu a précipité. Il a été filtré et séché sous vide pour donner un solide avec un rendement de 40%.

L'aspartame a donc été obtenu sous forme pure, sans utiliser de solvants organiques, et sans produits secondaires organiques. L'unique étape de purification a consisté en une précipitation dans de l'eau pour obtenir de l'aspartame solide.

## Revendications

1. Procédé de synthèse d'un composé de formule (I) suivante : 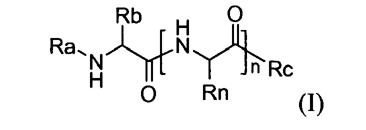 dans laquelle :
- n est un entier supérieur ou égal à 1, de préférence compris entre 1 et 100, plus préférentiellement compris entre 1 et 50, plus préférentiellement encore égal à 1 ou 2;
- Rb et chaque Rn représentent indépendamment les uns des autres un atome d'hydrogène, un groupe aryle(alkyle en C₁ à C₆) ou un groupe alkyle en C₁ à C₆ substitué ou non par un groupe aryle, -COOH, -COO-(alkyle en C₁ à C₆), -CONH₂, - SH, hétéroaryle, -NH₂, -NHC(NH)(NH₂), -S-(alkyle en C₁ à C₆), -OH ou phénol dont les fonctions NH₂, NH, COOH, SH et OH de ces groupes sont avantageusement protégés par un ou plusieurs groupe O-protecteur et/ou N-protecteur identiques ou différents et différent de Ra;
- Ra représente un groupe N-protecteur ;
- Rc représente un groupe -ORd dans lequel Rd représente un groupe alkyle en C₁ à C₆ ou un groupe -NReRf dans lequel Re et Rf représentent indépendamment l'un de l'autre un groupe N-protecteur,
**caractérisé en ce qu'**il comprend une étape (a) consistant à faire réagir en présence d'une base et sans solvant le composé de formule (II) suivante : 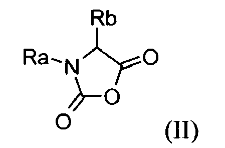 dans laquelle Ra et Rb sont tels que définis précédemment, avec le composé de formule (III) suivante : 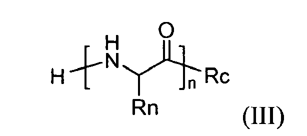 dans laquelle n, Rn et Rc sont tels que définis précédemment, ainsi que ses sels pharmaceutiquement acceptables, de préférence les chlorures, acétates et trifluoroacétates.

2. Procédé selon la revendication 1, **caractérisé en ce que** n est égal à 1 et **en ce que** l'étape (a) consiste à faire réagir en présence d'une base et sans solvant le composé de formule (II) avec le composé de formule (III-1) suivante : 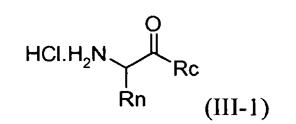 dans laquelle R1 et Rc sont tels que définis précédemment de façon à obtenir le composé de formule (I-1) suivante : 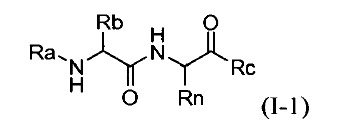 dans laquelle Ra, Rb, Rc et Rn sont tels que définis précédemment.

3. Procédé selon l'une quelconque des revendications précédentes, **caractérisé en ce que** l'étape (a) est effectuée au moyen d'un broyage à billes.

4. Procédé selon l'une quelconque des revendications précédentes, **caractérisé en ce que** la base est choisie dans le groupe constitué par les carbonates, de préférence les hydrogénocarbonates de sodium, de potassium et de césium, et les carbonates de sodium, de potassium et de césium.

5. Procédé selon l'une quelconque des revendications précédentes, **caractérisé en ce que** Rb et chaque Rn sont choisis indépendamment dans le groupe constitué par l'hydrogène, -CH₃, le groupe benzyle, -CH₂CONH₂, -CHCH₃C₂H₅, -(CH₂)₃NHC(NH)(NH₂), -CH(OH)CH₃, -CH₂COOH, -CH₂SH, -CH₂CH₂COOH, -CH₂CH₂CONH₂, imidazolylméthyle, propyle, -CH₂CH(CH₃)₂, -(CH₂)₄NH₂, -(CH₂)₂SCH₃, -CH₂OH, indol-2-ylméthyle, *p*-méthylphénol, isopropyle, les fonctions NH₂, NH, COOH, SH et OH de ces groupes étant avantageusement protégés par un ou plusieurs groupe O-protecteur et/ou N-protecteur identiques ou différents et différent de Ra.

6. Procédé selon l'une quelconque des revendications 1 à 4, **caractérisé en ce que** :
- Ra, Re et Rf sont choisis indépendamment l'un de l'autre dans le groupe constitué par le *tert-*butyloxycarbonyle, le 9-fluorénylméthyloxycarbonyle, le benzyloxycarbonyle, le nitroveratryloxycarbonyle ;
- Rb est choisi dans le groupe constitué par l'isopropyle, le benzyle et -CH₂COOt-Bu ;
- chaque Rn est choisi indépendamment des autres dans le groupe constitué par le méthyle, le benzyle et -CH₂CH(CH₃)₂ ;
- Rd est choisi dans le groupe constitué par le méthyle et le tertiobutyle.

7. Procédé selon l'une quelconque des revendications 2 à 6, **caractérisé en ce que** :
- le composé (II) est choisi dans le groupe constitué par les composés de formules (II-a), (11-b) et (II-c) suivantes : 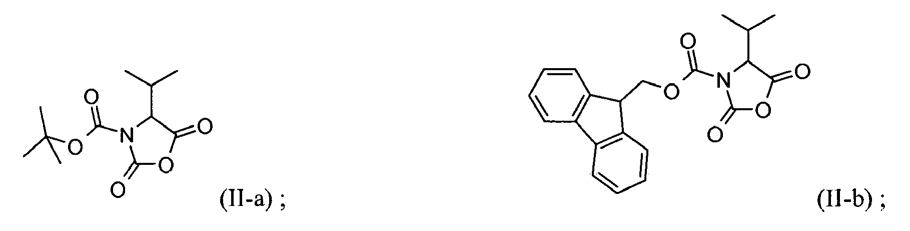 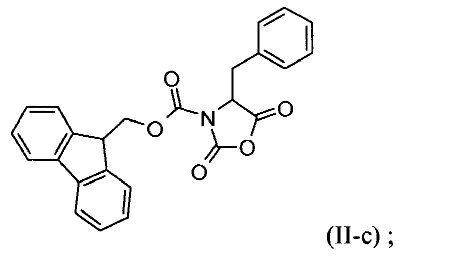
- le composé (III) est choisi dans le groupe constitué par les composés de formules (III-a), (III-b), (III-c), (III-d) et (III-e) suivantes :

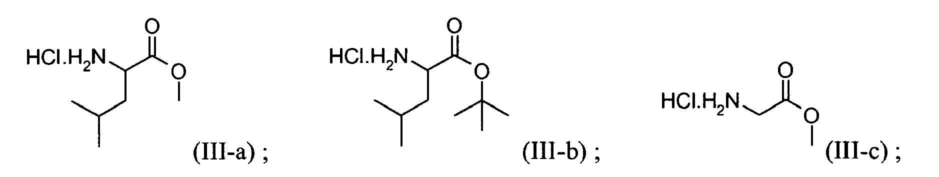

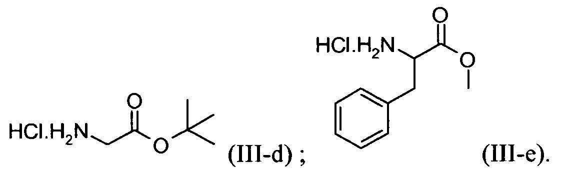

8. Procédé selon la revendication 2, **caractérisé en ce que** :
- le composé de formule (II) a la formule (II-2) suivante : 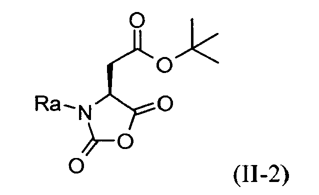 dans laquelle Ra est tel que défini à la revendication 1,
- le composé de formule (III) a la formule (III-2) suivante : 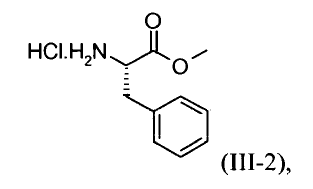 et le composé de formule (I) a la formule (I-2) suivante : 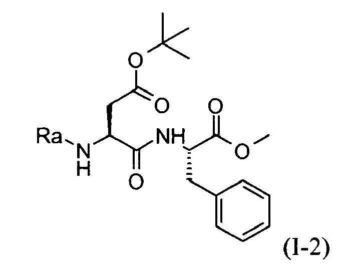 dans laquelle Ra est tel que défini à la revendication 1.

9. Procédé selon la revendication 8, **caractérisé en ce que** le composé de formule (II-2) est préparé par un procédé comprenant les étapes successives suivantes :
1) protection du groupe amine du composé de formule (IV) suivante : 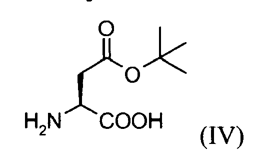 par un groupement N-protecteur Ra pour former le composé de formule (V) suivante 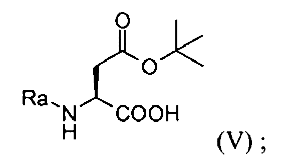
2) réaction du composé de formule (V) avec un halogénure de benzyle en présence d'une base pour obtenir le composé de formule (VI) suivante : 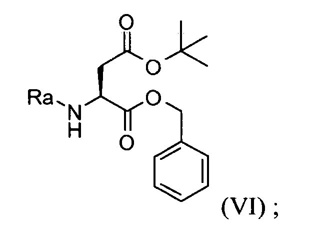 dans laquelle Ra est tel que défini ci-dessus
3) protection du groupe amine du composé de formule (VI) par un groupe N-protecteur Rg pour former le composé de formule (VII) suivante : 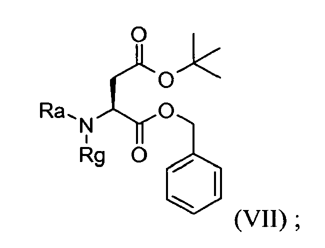 dans laquelle Ra et Rg sont tels que définis ci-dessus
4) réduction du composé de formule (VII) en composé de formule (VIII) suivante : 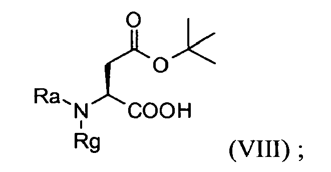 dans laquelle Ra et Rg sont tels que définis ci-dessus
5) cyclisation du composé de formule (VIII) en composé de formule (II-2) par réaction avec la DMF et le chlorure d'oxalyle.

10. Procédé selon la revendication 9, **caractérisé en ce que** Ra et Rg représentent le *tert*-butyloxycarbonyle.

11. Procédé selon l'une quelconque des revendications 8 à 10, **caractérisé en ce qu'**il comprend les étapes successives supplémentaires suivantes :
- (b) faire réagir le composé de formule (I-2) obtenu à l'étape (a) avec un acide pour former le composé de formule (I-3) suivante : 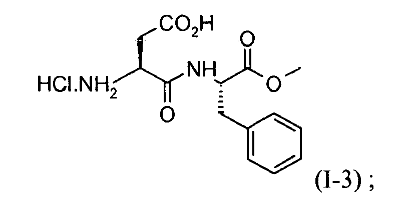
- (c) faire réagir le composé de formule (I-3) avec une base pour former le composé de formule (I-4) suivante :

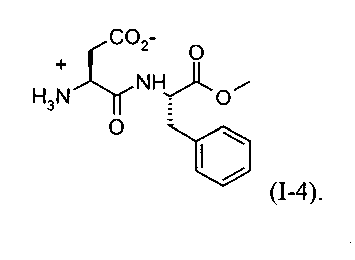

## Claims

1. A method for synthesizing a compound of the following formula (I) 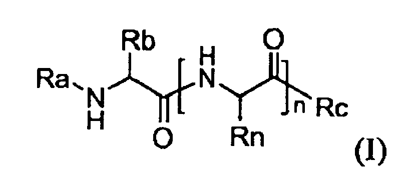 wherein:
- n is an integer larger than or equal to 1, preferably comprised between 1 and 100, more preferentially comprised between 1 and 50, still more preferentially equal to 1 or 2;
- Rb and each Rn represent independently of each other a hydrogen atom, an aryl(C₁-C₆ alkyl) group, or a C₁-C₆ alkyl group either substituted or not by an aryl group, -COOH, -COO-(C₁-C₆ alkyl), -CONH₂, -SH, heteroaryl, -NH₂, -NHC (NH) (NH₂), -S- (C₁-C₆ alkyl), -OH or phenol, the functions of which NH₂, NH, COOH, SH, OH are advantageously protected by one or more identical or different N-protective and/or O-protective groups and different from the Ra;
- Ra represents an N-protective group;
- Rc represents an -ORd group wherein Rd represents a C₁-C₆ alkyl group or an -NReRf group wherein Re and Rf represent independently of each other an N-protective group,
**characterized in that** it comprises a step (a) consisting of reacting in the presence of a base and without any solvent, the compound of the following formula (II): 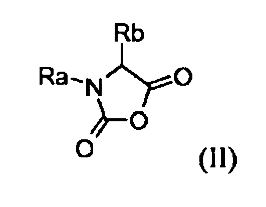 wherein Ra and Rb are as defined earlier, with the compound of the following formula (III): 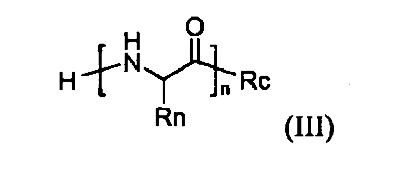 wherein n, Rn and Rc are as defined earlier, as well as its pharmaceutically acceptable salts, preferably chlorides, acetates and trifluoroacetates.

2. The method according to claim 1, **characterized in that** n is equal to 1 and **in that** step (a) consists of reacting in the presence of a base and without any solvent the compound of formula (II) with the compound of the following formula (III-1): 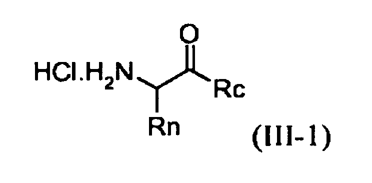 wherein R1 and Rc are as defined earlier so as to obtain the compound of the following formula (I-1): 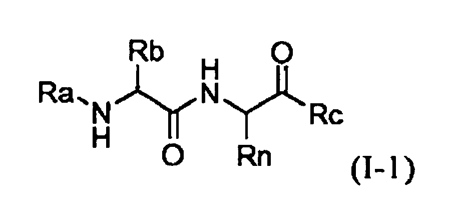 wherein Ra, Rb, Rc and Rn are as defined earlier.

3. The method according to any of the preceding claims, **characterized in that** step (a) is carried out by means of ball-milling.

4. The method according to any of the preceding claims, **characterized in that** the base is selected from the group consisting of carbonates, preferably, sodium, potassium and cesium hydrogencarbonates, and sodium, potassium and cesium carbonates.

5. The method according to any of the preceding claims, **characterized in that** Rb and each Rn are independently selected from the group consisting of hydrogen, -CH₃, the benzyl group, -CH₂CONH₂, -CHCH₃C₂H₅, - (CH₂) ₃NHC (NH) (NH₂), -CH (OH) CH₃, -CH₂COOH, -CH₂SH, - CH₂CH₂COOH, -CH₂CH₂CONH₂, imidazolylmethyl, propyl, - CH₂CH (CH₃)₂, - (CH₂) ₄NH₂, -(CH₂) ₂SCH₃, -CH₂OH, indol-2-ylmethyl, *p*-methylphenol, isopropyl groups, the NH₂, NH, COOH, SH and OH functions of these groups being advantageously protected by one or more identical or different O-protective and/or N-protective groups and different from Ra.

6. The method according to any of claims 1 to 4, **characterized in that**
- Ra, Re and Rf are selected independently of each other from the group consisting of *tert-*butyloxycarbonyl, 9-fluorenylmethyloxycarbonyl, benzyloxycarbonyl, nitro-veratryloxycarbonyl;
- Rb is selected from the group consisting of isopropyl, benzyl and -CH₂COOt-Bu;
- each Rn is selected independently of the others from the group consisting of methyl, benzyl and - CH₂CH(CH₃)₂ ;
- Rd is selected from the group consisting of methyl and tertiobutyl.

7. The method according to any of claims 2 to 6, **characterized in that**:
- the compound (II) is selected from the group consisting of the compounds of the following formulae (II-a), (II-b) and (IIc) : 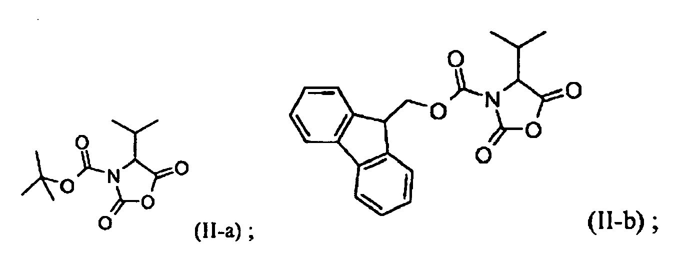 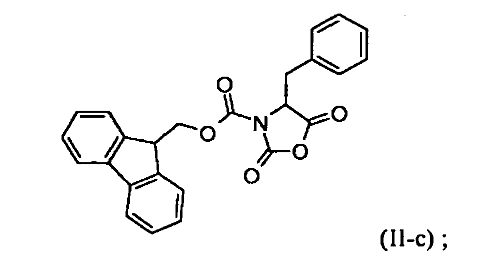
- the compound (III) is selected from the group consisting of the compounds of the following formulae (III-a), (III-b),(III-c),(III-d) and (III-e) :

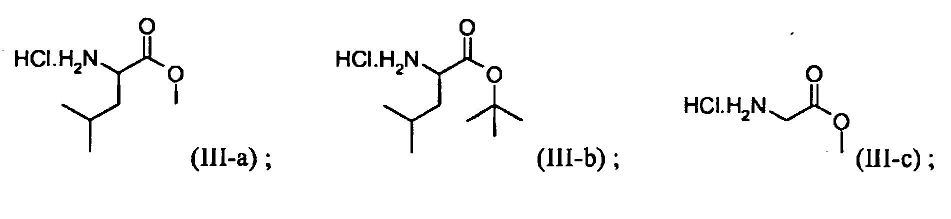

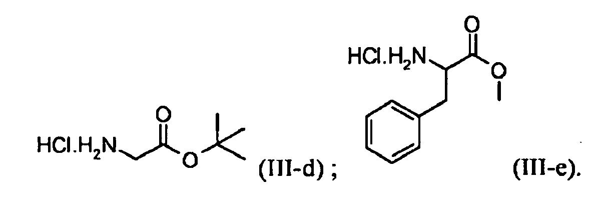

8. The method according to claim 2, **characterized in that**:
- the compound of formula (II) has the following formula (II-2): 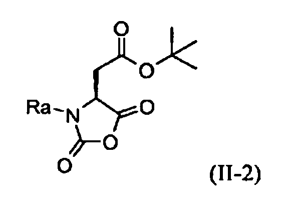 wherein Ra is as defined in claim 1,
- the compound of formula (III) has the following formula (III-2): 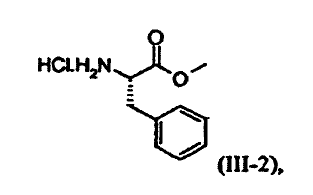 and the compound of formula (I) has the following formula (I-2): 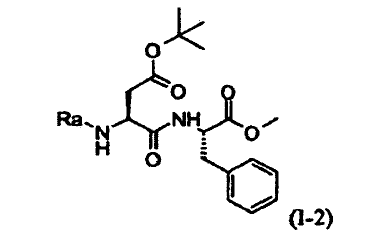 wherein Ra is as defined in claim 1.

9. The method according to claim 8, **characterized in that**
the compound of formula (II-2) is prepared by a method comprising the following successive steps:
1) protecting the amine group of the compound of the following formula (IV): 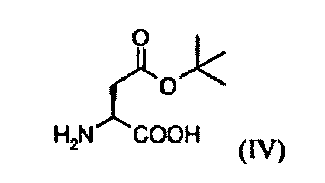 with an N-protective group Ra in order to form the compound of the following formula (V): 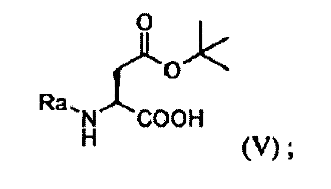
2) reacting the compound of formula (V) with a benzyl halide in the presence of a base in order to obtain the compound of the following formula (VI): 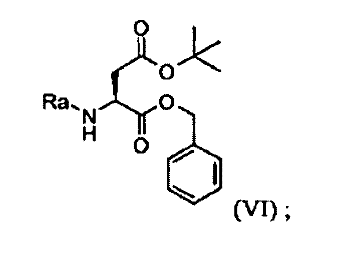 wherein Ra is as defined above;
3) protecting the amine group of the compound of formula (VI) with an N-protective group Rg in order to form the compound of the following formula (VII): 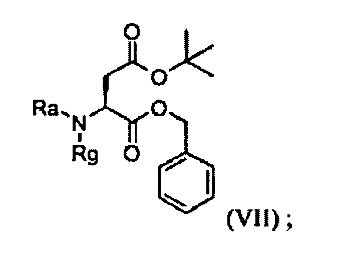 wherein Ra and Rg are as defined above;
4) reducing the compound of formula (VII) into a compound of the following formula (VIII): 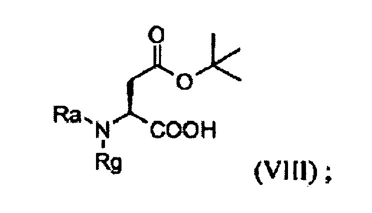 wherein Ra and Rg are as defined above;
5) cyclizing the compound of formula (VIII) into a compound of formula (II-2) by reaction with DMF and oxalyl chloride.

10. The method according to claim 9, **characterized in that** Ra and Rg represent *tert-*butyloxycarbonyl.

11. The method according to any of claims 8 to 10, **characterized in that** it comprises the following additional successive steps:
- (b) reacting the compound of formula (I-2) obtained in step (a) with an acid in order to form the compound of the following formula (I-3): 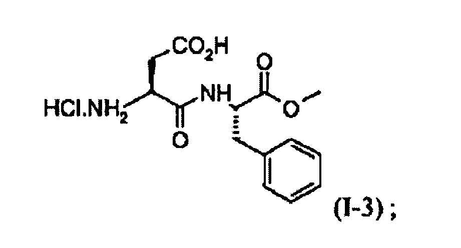
- (c) reacting the compound of formula (I-3) with a base in order to form the compound of the following formula (I-4):

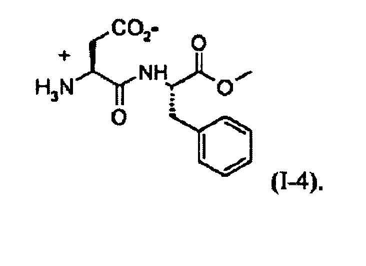

## Patentansprüche

1. Verfahren zur Synthese einer Verbindung der folgenden Formel (I): 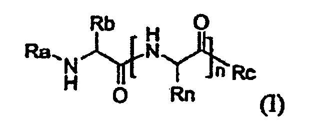 in der:
- n eine ganze Zahl größer oder gleich 1, vorzugsweise zwischen 1 und 100, stärker bevorzugt zwischen 1 und 50, noch stärker bevorzugt gleich 1 oder 2 ist;
- Rb und jedes Rn unabhängig voneinander ein Wasserstoffatom, einen Aryl(C₁-C₆-alkyl)rest oder einen C₁-C₆-Alkylrest, unsubstituiert oder substituiert mit einem Arylrest, -COOH, -COO-(C₁-C₆-Alkyl), - CONH₂, -SH, Heteraryl, -NH₂, NHC(NH)(NH₂), -S-(C₁-C₆-Alkyl), -OH oder Phenol, bedeuten, wobei die Funktionen NH₂, NH, COOH, SH und OH dieser Reste vorteilhafterweise durch eine oder mehrere identische oder verschiedene und sich von Ra unterscheidende O-Schutzgruppen und/oder N-Schutzgruppen geschützt sind;
- Ra eine N-Schutzgruppe bedeutet;
- Rc einen Rest -ORd bedeutet, in dem Rd einen C₁-C₆-Alkylrest oder einen Rest -NReRf bedeutet, in dem Re und Rf unabhängig voneinander eine N-Schutzgruppe bedeuten,
**dadurch gekennzeichnet, dass** es einen Schritt (a) des Umsetzenlassens, in Gegenwart einer Base und ohne Lösungsmittel, der Verbindung der folgenden Formel (II): 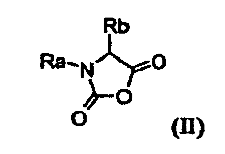 in der Ra und Rb wie vorstehend definiert sind, mit der Verbindung der folgenden Formel (III) umfasst: 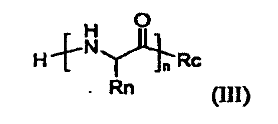 in der n, Rn und Rc wie vorstehend definiert sind, sowie ihrer pharmazeutisch verträglichen Salze, vorzugweise Chloride, Acetate und Trifluoracetate.

2. Verfahren nach Anspruch 1, **dadurch gekennzeichnet, dass** n gleich 1 ist und dass Schritt (a) darin besteht, die Verbindung der Formel (II) in Gegenwart einer Base und ohne Lösungsmittel mit der Verbindung der folgenden Formel (III-1) reagieren zu lassen: 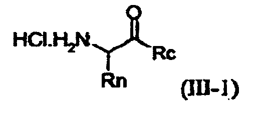 in der R1 und Rc wie vorstehend definiert sind, um die Verbindung der folgenden Formel (I-1) zu erhalten: 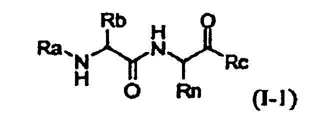 in der Ra, Rb, Rc und Rn wie vorstehend definiert sind.

3. Verfahren nach einem der vorhergehenden Ansprüche, **dadurch gekennzeichnet, dass** Schritt (a) mittels einer Zerkleinerung zu Kügelchen durchgeführt wird.

4. Verfahren nach einem der vorhergehenden Ansprüche, **dadurch gekennzeichnet, dass** die Base aus der Gruppe bestehend aus Carbonaten, vorzugweise Natrium-, Kalium- und Cäsiumhydrogencarbonaten, und Natrium-, Kalium- und Cäsiumcarbonaten ausgewählt ist.

5. Verfahren nach einem der vorhergehenden Ansprüche, **dadurch gekennzeichnet, dass** Rb und jedes Rn unabhängig aus der Gruppe bestehend aus Wasserstoff, -CH₃, Benzylrest, -CH₂CONH₂, -CHCH₃C₂H₅, -(CH₂)₃NHC(NH)(NH₂), -CH(OH)CH₃, - CH₂COOH, -CH₂SH, -CH₂CH₂COOH, -CH₂CH₂CONH₂, Imidazolylmethyl, Propyl, -CH₂CH(CH₃)₂, -(CH₂)₄NH₂, -(CH₂)₂SCH₃, -CH₂OH Indol-2-ylmethyl, p-Methylphenol, Isopropyl ausgewählt sind, wobei die Funktionen NH₂, NH, COOH, SH und OH dieser Reste vorteilhafterweise durch eine oder mehrere identische oder verschiedene und sich von Ra unterscheidende O-Schutzgruppen und/oder N-Schutzgruppen geschützt sind.

6. Verfahren nach einem der Ansprüche 1 bis 4, **dadurch gekennzeichnet, dass**:
- Ra, Re und Rf unabhängig voneinander aus der Gruppe bestehend aus tert-Butyloxycarbonyl, 9-Fluorenylmethyloxycarbonyl, Benzyloxycarbonyl, Nitroveratryloxycarbonyl ausgewählt sind;
- Rb aus der Gruppe bestehend aus Isopropyl, Benzyl und -CH₂COOt-Bu ausgewählt ist;
- jedes Rn unabhängig voneinander aus der Gruppe bestehend aus Methyl, Benzyl und -CH₂CH(CH₃)₂ ausgewählt ist;
- Rd aus der Gruppe bestehend aus Methyl und tert-Butyl ausgewählt ist.

7. Verfahren nach einem der Ansprüche 2 bis 6, **dadurch gekennzeichnet, dass**:
- die Verbindung (II) aus der Gruppe bestehend aus den Verbindungen der folgenden Formeln (II-a), (II-b) und (II-c) ausgewählt ist: 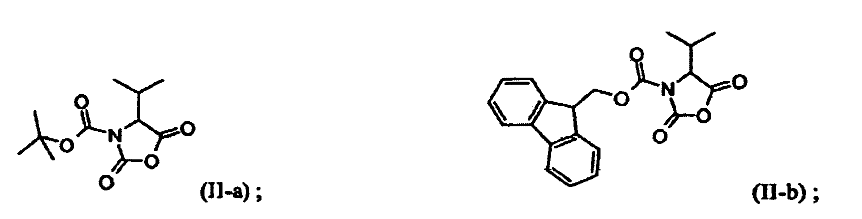 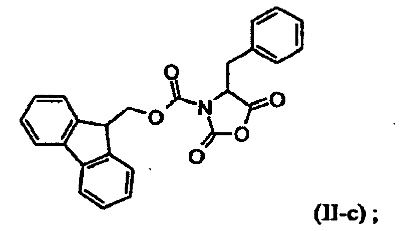
- die Verbindung (III) aus der Gruppe bestehend aus den Verbindungen der folgenden Formeln (III-a), (III-b), (III-c), (III-d) und (III-e) ausgewählt ist:

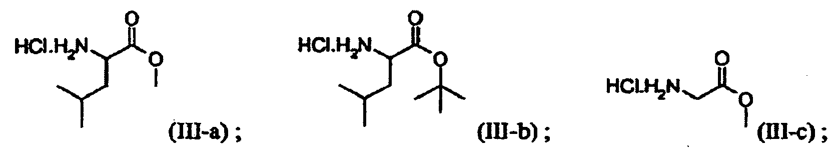

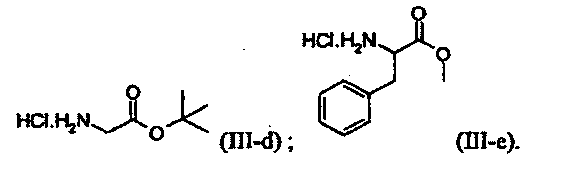

8. Verfahren nach Anspruch 2, **dadurch gekennzeichnet, dass**:
- die Verbindung der Formel (II) die folgende Formel (11-2) aufweist: 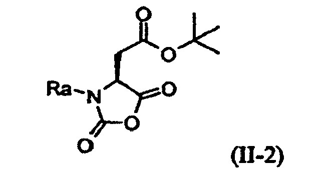 in der Ra wie in Anspruch 1 definiert ist,
- die Verbindung der Formel (III) die folgende Formel (III-2) aufweist: 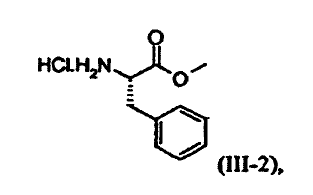 und die Verbindung der Formel (I) die folgende Formel (I-2) aufweist: 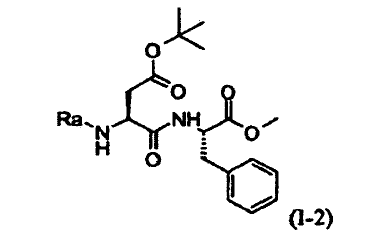 in der Ra wie in Anspruch 1 definiert ist.

9. Verfahren nach Anspruch 8, **dadurch gekennzeichnet, dass** die Verbindung der Formel (II-2) durch ein Verfahren hergestellt wird, das die folgenden aufeinanderfolgenden Schritte umfasst:
1) Schützen der Amingruppe der Verbindung der folgenden Formel (IV): 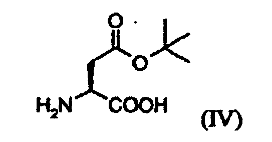 mit einer N-Schutzgruppe Ra, um die Verbindung der folgenden Formel (V) zu bilden: 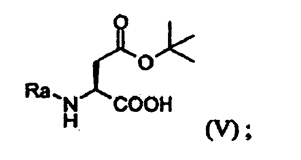
2) Umsetzen der Verbindung der Formel (V) mit einem Benzylhalogenid in Gegenwart einer Base, um die Verbindung der folgenden Formel (VI) zu erhalten: 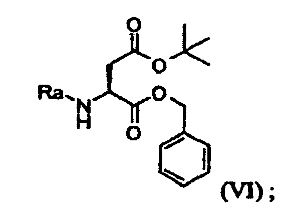 in der Ra wie vorstehend definiert ist;
3) Schützen der Amingruppe der Verbindung der Formel (VI) durch eine N-Schutzgruppe Rg, um die Verbindung der folgenden Formel (VII) zu bilden: 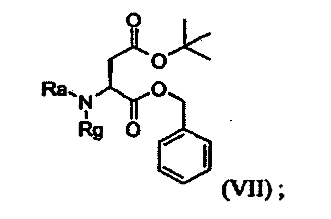 in der Ra und Rg wie vorstehend definiert sind;
4) Reduktion der Verbindung der Formel (VII) zur Verbindung der folgenden Formel (VIII): 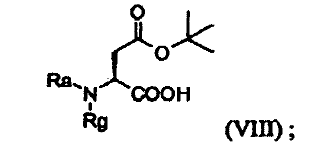 in der Ra und Rg wie vorstehend definiert sind,
5) Ringschluss der Verbindung der Formel (VIII) zur Verbindung der Formel (II-2) durch Reaktion mit DMF und Oxalylchlorid.

10. Verfahren nach Anspruch 9, **dadurch gekennzeichnet, dass** Ra und Rg tert-Butyloxycarbonyl bedeuten.

11. Verfahren nach einem der Ansprüche 8 bis 10, **dadurch gekennzeichnet, dass** es die folgenden zusätzlichen aufeinanderfolgenden Schritte umfasst:
- (b) Umsetzenlassen der in Schritt (a) erhaltenen Verbindung der Formel (I-2) mit einer Säure, um die Verbindung der folgenden Formel (I-3) zu bilden: 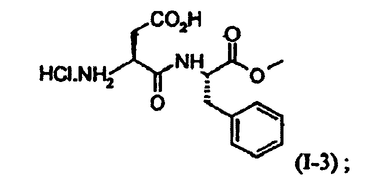
- (c) Umsetzenlassen der Verbindung der Formel (1-3) mit einer Base, um die Verbindung der folgenden Formel (I-4) zu bilden:
